# EUROPÄISCHE PATENTANMELDUNG

(11) **EP 3 718 589 A1**
(43) Veröffentlichungstag der Anmeldung: **07.10.2020**
(21) Anmeldenummer: 20165513.1
(22) Anmeldetag: 25.03.2020
(51) Int. Cl.: A61M 25/00, A61M 25/10

(54) **BLASENKATHETER UND VERFAHREN ZUR HERSTELLUNG EINES BLASENKATHETERS**

(30) Priorität: 26.03.2019 DE 102019107707
(71) Anmelder: Saxonia R + D GmbH & Co. KG, 01454 Radeberg (DE)
(72) Erfinder: Lüning, Rudolph, 01099 Dresden (DE); Purcell, Larry, 01454 Radeberg (DE); Wirth, Manfred, 01326 Dresden (DE)
(74) Vertreter: Kailuweit & Uhlemann Patentanwälte Partnerschaft mbB

(57) **Zusammenfassung**

Die Erfindung betrifft Blasenkatheter jeweils mit einem Schlauch mit einer Öffnung am distalen Ende und mindestens einem in der Harnblase zu platzierenden Katheterauge, wobei der Schlauch wenigstens einen gegenüber einem Wandbereich des Schlauchs aufblähbaren oder befüllbaren Wandbereich aufweist. Diese zeichnen sich insbesondere dadurch aus, dass diese als Dauerkatheter mit einem sicheren Halt in der Blase und zu deren vollständigen Entleerung einsetzbar sind. Dazu besteht der Blasenkatheter aus mehreren nacheinander aufgebrachten Schichten eines gummiartigen Stoffes. Der Wandbereich ist wenigstens ein Wandbereich des Schlauchs, wobei der Wandbereich wenigstens zwei voneinander getrennte Schichten des gummiartigen Stoffes des Wandbereichs des Schlauchs sind. Ein Bestandteil der Wand des Schlauchs ist wenigstens ein Kanal zum Aufblähen und/oder Befüllen und/oder Reinigen des Wandbereichs, so dass der Schlauch einstückig ausgebildet ist und wobei der Schlauch die miteinander verbundenen Schichten, den wenigstens einen Wandbereich mit den voneinander getrennten Schichten und den Kanal aufweist.

## Beschreibung

Die Erfindung betrifft Blasenkatheter jeweils mit einem Schlauch mit einer Öffnung am distalen Ende und mindestens einem in der Harnblase zu platzierenden Katheterauge, wobei der Schlauch wenigstens einen gegenüber einem Wandbereich des Schlauchs aufblähbaren oder befüllbaren Wandbereich aufweist, und Verfahren zu deren Herstellung.

Blasenkatheter werden in der Medizin zum Ablassen von Harn (Urin) aus der Harnblase über die Harnröhre (transurethral) oder die Bauchdecke verwendet. Dabei wird zwischen Einmakatheter und Dauerkatheter unterschieden. Letztere sind für einen längeren Verbleib in der Harnröhre und/oder Harnblase konzipiert und dienen der kontinuierlichen oder vorübergehenden Ableitung von Urin aus der Harnblase. Katheter kommen nicht nur bei Eingriffen, die die Harnwege und/oder die Harnblase oder die Prostata betreffen zum Einsatz, sondern werden sehr oft postoperativ verwendet, wenn absehbar ist, dass der Patient für einige Zeit nicht aufstehen kann oder eine genaue Flüssigkeitsbilanzierung erforderlich ist.

Dauerkatheter sind in der Regel als Zwei-Wege-Katheter ausgestaltet, wobei der Katheterschlauch neben einem Kanal zum Ablassen des Urins einen zweiten Kanal aufweist, über den ein am Katheterende befindlicher Ballon in der Harnblase mit Flüssigkeit gefüllt werden kann. Dieser dient als Halter des Blasenkatheters in der Harnblase. Als Flüssigkeit zum Füllen wird vorrangig Kochsalzlösung oder Glycerin oder Glycerinlösung verwendet. Es kommen auch Drei-Wege-Katheter zum Einsatz, bei denen der Katheterschlauch einen dritten Kanal mit einer Austrittsöffnung am Katheterende aufweist, durch den beispielsweise eine Spüllösung oder ein Medikament in die Blase eingebracht werden kann. Die Spüllösung verlässt die Blase, wie der Urin, durch eine Öffnung, auch als Katheterauge bezeichnet, durch den Hauptkanal.

Die Druckschrift DE 20 2012 006 814 U1 beinhaltet einen Blasenkatheter mit einem Schlauch, wobei der Schlauch einen Einführabschnitt zum Einführen über eine Harnröhre in eine Harnblase aufweist und wobei der Schlauch einen Anschlussabschnitt zum Anschließen von Anschlusselementen aufweist und wobei durch den Einführabschnitt Fluid aus der Harnröhre am Anschlussabschnitt ablassbar ist. Dem Anschlussabschnitt ist ein verschließbares Ventil derart angeordnet, dass ein Austreten von Fluid aus dem Anschlussabschnitt bei verschlossenem Ventil verhindert werden kann. So kann bei verschlossenem Ventil ein voller Urinbeutel gewechselt werden ohne dass Urin aus dem Anschlussabschnitt des Blasenkatheters austreten kann. Durch die Druckschrift DE 20 2007 007 713 U1 ist ein Mehrwegekatheter bekannt, der sich von den bekannten Katheter dadurch unterscheidet, dass der Katheterkörper mindestens einen dritten Kanal zur Applikation von mindestens einem Medikament parallel zum Katheterkörper mit mindestens einer Austrittsöffnung aufweist. Durch diese Austrittsöffnung kann nach Einführen des Katheters in die Harnblase eine gezielte Applikation von Medikamenten in dem Bereich der Harnröhre über den dritten Kanal des Katheters stattfinden.

Ein transurethraler Verweilkatheter zur Behandlung von Funktionsstörungen der Blase und/oder der Prostata ist durch die Druckschrift DE 20 2011 104 675 U1 bekannt. Der Verweilkatheter besteht aus einem Katheterschlauch, einem Blasen- und einem Prostataballon mit entsprechenden Zu- und Ableitungen. Dabei ist die Urinablauföffnung oberhalb und anatomisch bedingt der Prostataballon unterhalb des Blasenballons angeordnet. Die Wandungen von Blasen- und Prostataballon sind so ausgestaltet, dass in den Ballons gespeicherte, Medikamente enthaltende Flüssigkeiten gezielt in die Blase oder die Prostata diffundieren können. Der Prostataballon weist im befülltem Zustand einen maximalen Außendurchmesser zwischen 20 und 30 Charr. auf. Der Verweilkatheter soll eine maximale Verweildauer von 48h in der Blase haben. Nachteilig dient dieser Katheter ausschließlich der Anwendung beim Mann. Außerdem ist davon auszugehen, dass die offenbarte Ballonstärke wesentlich zu gering gewählt ist, um den Katheter in der Blase zu halten.

Die Druckschrift WO 2017/174 715 A1 offenbart einen verbesserten Blasenkatheter mit einem Katheterschlauch mit einem ersten Anschluss am distalen Ende und mindestens einem proximalen Katheterauge, der auch als Dauerkatheter verwendbar ist. In der Harnblase befindet sich dazu ein Reservoirbehälter, der über einen Kanal mit einem zweiten Anschluss als distales Ende des Kanals verbunden ist. Weiterhin ist der Kanal über wenigstens eine Öffnung der Kanalwand mit der Harnröhre verbunden. Der Blasenkatheter kann über die Harnröhre in die Harnblase (transurethal) oder als suprapubischer Blasenkatheter über den Bauch in die Harnblase eingeführt werden. Ausführungen zur Realisierung der ballonförmigen Halteelemente, Reservoirbehälter und Kanäle sind nicht Gegenstand dieser Druckschrift.

Die Druckschrift WO 96 26 748 A2 betrifft Ballonkatheter, mit einen aufblasbaren Ballon. Dieser wird nach dem Formungsprozess distal gefaltet, bis das distale Ende an den sich nach innen verjüngenden Hals des Katheterkörpers angrenzt. Unter Verwendung einer Klebeverbindung werden diese miteinander verbunden. Das Aufblaslumen kann durch Einführen eines entfernbaren Drahtes in die Form entlang des vorbestimmten Weges des Aufblaslumens geformt werden. Damit kann das Aufblaslumen somit ein intgraler Bestandteil der Katheterwand sein. Die Druckschrift DE 25 40 385 A1 offenbart einen Katheter zur Entnahme von Urin aus der Blase eines Patienten, der einen aufblasbaren Ballon besitzt. Dieser kann hergestellt werden, indem die Außenfläche des Schaftes des Katheters mit Betonit oder einer anderen wasserlöslichen, sich trennenden Masse während der Herstellung des Katheters beschichtet wird und der beschichtete Schaft in Gummimilch eingetaucht wird. Zum Aufblasen des Ballons besitzt der Katheter eine Füllkanal. Auf dessen Realisierung wird nicht näher eingegangen.

Durch die Druckschrift WO 2005 023 153 A2 ist ein Ballonkathetersystem bekannt, der mehrere Ballone aufweisen kann, die ineinander angeordnet sind. Diese können mit verschiedenen Medien befüllt werden. Damit kann eine gesteuerte Expansion ermöglicht werden, um Stents verschiedener Länge und Durchmesser zu setzen.

Der in den Patentansprüchen 1 und 10 angegebenen Erfindung liegt die Aufgabe zugrunde, einen Blasenkatheter als Dauerkatheter mit einem sicheren Halt in der Blase und zu deren vollständigen Entleerung bereitzustellen.

Diese Aufgabe wird mit den in den Patentansprüchen 1 und 11 aufgeführten Merkmalen gelöst.

Die Blasenkatheter jeweils mit einem Schlauch mit einer Öffnung am distalen Ende und mindestens einem in der Harnblase zu platzierenden Katheterauge, wobei der Schlauch wenigstens einen gegenüber einem Wandbereich des Schlauchs aufblähbaren oder befüllbaren Wandbereich aufweist, zeichnen sich insbesondere dadurch aus, dass diese als Dauerkatheter mit einem sicheren Halt in der Blase und zu deren vollständigen Entleerung einsetzbar sind

Dazu besteht der Blasenkatheter aus mehreren nacheinander aufgebrachten Schichten eines gummiartigen Stoffes. Der Wandbereich ist wenigstens ein Wandbereich des Schlauchs, wobei der Wandbereich wenigstens zwei voneinander getrennte Schichten des gummiartigen Stoffes des Wandbereichs des Schlauchs sind. Ein Bestandteil der Wand des Schlauchs ist wenigstens ein Kanal zum Aufblähen und/oder Befüllen und/oder Reinigen des Wandbereichs, so dass der Schlauch einstückig ausgebildet ist und wobei der Schlauch die miteinander verbundenen Schichten, den wenigstens einen Wandbereich mit den voneinander getrennten Schichten und den Kanal aufweist.

Der Blasenkatheter ist über die Harnröhre in die Harnblase (transurethal) oder als suprapubischer Blasenkatheter über den Bauch in die Harnblase einführbar. Der Schlauch ist dabei eine hohle und flexible Leitung durch die der Harn aus der Harnblase abgeleitet werden kann. Dazu besitzt dieser am distalen Ende die Öffnung.

Bei dem Blasenkatheter befinden sich die Öffnung des Schlauchs und eine Öffnung des Kanals am distalen Ende und damit dem dem Körper abgewandtem Ende des Schlauchs. Diese Öffnungen befinden sich bei platziertem Blasenkatheter außerhalb des Körpers. Die Öffnung des Schlauchs kann verschlossen und/oder mit einem Beutel zum Sammeln des Urins aus der Harnblase versehen sein. Die Öffnung des Kanals ist verschließbar, so dass mittels dem aufgeblähten Wandbereich ein sicherer Halt des Blasenkatheters gegeben ist.

Die Länge des Schlauchs des Blasenkatheters kann individuell gewählt werden. Ist der Blasenkatheter für die Anwendung bei einem Mann vorgesehen, so ist der Schlauch länger ausgestaltet als bei einem Blasenkatheter für eine Frau.

Das Katheterauge ist eine Öffnung in der Wand des Schlauchs, durch die nach dem Einführen des Blasenkatheters in die Harnblase Urin aus der Harnblase über den Schlauch zur ersten Öffnung gelangen kann. Das Katheterauge kann eine runde bis ovale Form besitzen.

Vorteilhafterweise besteht der Blasenkatheter aus mehreren nacheinander aufgebrachten Schichten eines gummiartigen Stoffes, wobei der aufblähbare Wandbereich wenigstens ein Wandbereich des Schlauchs ist, der durch ein entfernbares Trennmittel voneinander getrennte Schichten aufweist. Damit besitzt der Blasenkatheter über seine Länge den gleichen Umfang. Insbesondere Erhebungen sind nicht vorhanden, so dass eine einfache Platzierung des Blasenkatheters gegeben ist.

Zur Herstellung eines Blasenkatheters mit einem Schlauch mit einer Öffnung am distalen Ende und mindestens einem in der Harnblase zu platzierenden Katheterauge, wobei der Schlauch wenigstens einen gegenüber einem Wandbereich des Schlauchs aufblähbaren oder befüllbaren Wandbereich aufweist, können beispielsweise wenigstens eine Schicht eines polymerisierbaren und/oder vulkanisierbaren Stoffes auf einen Stützkörper, auf einem Wandbereich der Schicht ein entfernbares Trennmittel, auf die Schicht und das Trennmittel wenigstens eine weitere Schicht eines polymerisierbaren und/oder vulkanisierbaren Stoffes aufgebracht werden. Nach jedem Auftrag polymerisieren und/oder vulkanisieren die Schichten zu einem gummiartigen Stoff Das Trennmittel kann dazu eine Folie und/oder ein Pulver und/oder eine Schicht eines Stoffes sein. Das Aufbringen der Schichten kann durch Tauchen erfolgen, wobei zum Erreichen des wenigstens einen aufblähbaren oder befüllbaren Wandbereichs und einer gewünschten Dicke des Schlauchs ein mehrfaches Tauchen in den flüssigen und sich durch Polymerisation und/oder Vulkanisation ausbildenden gummiartigen Stoffes erfolgt.

Weiterhin zeichnet sich der Blasenkatheter dadurch aus, dass der aufgeblähte oder befüllte Wandbereich ein den Schlauch wenigstens bereichsweise nicht vollständig umgebender Ballon in der Harnblase *ist.* Der nicht durch den Ballon überdeckte Bereich des Schlauchs weist das Katheterauge auf. Der aufgeblähte oder befüllte Wandbereich als Ballon oder Wulst kann als offener Ring mit dem Querschnitt einer C-Form ausgebildet sein. Der aufgeblähte oder befüllte Wandbereich dient als Halter des Schlauchs. Im aufgeblähten oder befüllten Zustand ist somit der Ballon als Wulst am Schlauch vorhanden, der das Katheterauge in der Harnblase hält. Der Ballon umgibt den Schlauch nicht vollständig. In dem so nicht mit dem Ballon bedecktem Bereich des Schlauchs ist das Katheterauge angeordnet. Vorteilhafterweise kann das weitestgehend unmittelbar über den Schließmuskel der Harnröhre sein, so dass die Harnblase vollständig entleerbar ist. Es verbleibt kein Urinsumpf mit sich auskristallisierenden Salzen. Damit wird verhindert, dass Kristalle zwischen Schlauch und Harnröhre wandern und dort für schmerzhafte Reibung und/oder als Nährboden für Bakterien dienen können.
Die Schichten des Schlauchs können beispielsweise aus Latex bestehen.

Ein Blasenkatheter mit einem Schlauch mit einer Öffnung am distalen Ende und mindestens einem in der Harnblase zu platzierenden Katheterauge kann mit den folgenden Schritten hergestellt werden:
- Aufbringen wenigstens einer ersten Schicht eines polymerisierbaren und/oder vulkanisierbaren Stoffes auf einen Stützkörper,
- Polymerisieren und/oder Vulkanisieren der Schicht zu einem einseitig verschlossenen Schlauch eines gummiartigen Stoffs,
- Aufbringen eines entfernbaren Trennmittels auf einen Bereich des Schlauchs,
- Platzieren eines entfernbaren Fadens oder Garns oder Drahts, so dass dieser am oder auf oder im Trennmittel und außerhalb des Schlauchs als distales Ende endet,
- Aufbringen wenigstens einer zweiten Schicht eines polymerisierbaren und/oder vulkanisierbaren Stoffes auf den Schlauch, das entfernbare Trennmittel und den Faden oder das Garn oder den Draht,
- Polymerisieren und/oder Vulkanisieren der zweiten Schicht,
- Entfernen des Fadens oder Garns oder Drahts durch Herausziehen, so dass ein Kanal mit einer ersten Öffnung in Richtung aufblähbaren oder befüllbaren Wandbereich aus der zweiten Schicht über dem Trennmittel und einer zweiten Öffnung am distalen Ende des Schlauchs vorhanden ist, so dass der Wandbereich über den Kanal aufblähbar oder befüllbar ist,
- Entfernen des Schlauchs vom Stützkörper und
- Einbringen des Katheterauges in den Schlauch.

Vorteilhafte Ausgestaltungen der Erfindung werden nachfolgend angegeben.

Der aufgeblähte oder befüllte Wandbereich ist in einer Ausführungsform ein den Schlauch vollständig umgebende Wulst in der Harnröhre, wobei der Wandbereich wenigstens einen Durchbruch zur Harnröhre aufweist. Der aufgeblähte Wandbereich kann so ein Halter sein. Der Wandbereich kann mit einem flüssigen Medium befüllt sein, welches über den Durchbruch an die Innenwand der Harnröhre gelangen kann, die dadurch feucht haltbar ist. Das flüssige Medium kann wenigstens ein Medikament sein oder enthalten. Die Wulst ist so ein Reservoirbehälter.

Zwischen Schichten ist optional jeweils wenigstens ein entfernbarer Faden oder ein entfernbarer Draht oder ein entfernbares Garn so platziert, dass der Faden oder der Draht oder das Garn in den voneinander getrennten Schichten des Wandbereichs und außerhalb des distalen Endes endet und der Kanal ein durch Entfernen des Fadens oder des Drahts oder des Garns ausgebildeter Kanal ist. Dazu können der Faden ein Textil, Fasern oder Filamente, das Garn ein Textil oder der Draht ein Metalldraht und/oder Kunststoffdraht sein.

Aufblähbare oder befüllbare Wandbereiche sind in einer Ausführungsform gleichzeitig Wandbereiche des Schlauchs, wobei die Wandbereiche jeweils wenigstens drei voneinander getrennte Schichten des gummiartigen Stoffes des Wandbereichs des Schlauchs sind. Der Schlauch weist wenigstens zwei Kanäle zum Aufblähen oder Befüllen des jeweiligen Wandbereichs auf, so dass bei aufgeblähten oder befüllten Wandbereichen ein erster Ballon in einem zweiten Ballon jeweils im Querschnitt mit einer C-Form vorhanden sind und sich das Katheterauge im nicht durch die Ballone überdeckten Bereich des Schlauchs befindet. Weiterhin weist die in der Harnblase angeordnete Wand des zweiten Ballons wenigstens einen Durchbruch zur Harnblase auf. Der Wandbereich des zweiten Ballons weist den wenigstens einen Durchbruch auf. Dieser Durchbruch dient dem Austritt eines flüssigen Mediums, welches somit in die Harnblase in die Harnblase gelangt. Das flüssige Medium kann wenigstens ein Medikament sein oder enthalten. Der zweite Ballon ist so ein Reservoirbehälter.

Aufblähbare oder befüllbare Wandbereiche sind optional gleichzeitig Wandbereiche des Schlauchs, wobei die Wandbereiche wenigstens zwei voneinander getrennte Schichten des gummiartigen Stoffes des Wandbereichs des Schlauchs sind. Der Schlauch weist zwei Kanäle zum Aufblähen oder Befüllen eines ersten Wandbereichs und eines in Längsrichtung des Schlauchs beabstandet dazu angeordneten zweiten Wandbereichs auf, so dass bei aufgeblähten oder befüllten ersten Wandbereich ein Ballon im Querschnitt mit einer C-Form in der Harnblase und bei befüllten zweiten Wandbereich eine Wulst in der Harnröhre vorhanden sind, wobei sich das Katheterauge im nicht durch den Ballon überdeckten Bereich des Schlauchs befindet. Der Wandbereich der Wulst besitzt wenigstens einen Durchbruch zur Harnröhre, der dem Austritt eines flüssigen Mediums dient, welches somit in die Harnröhre gelangt. Das flüssige Medium kann wenigstens ein Medikament sein oder enthalten. Die Wulst ist so ein Reservoirbehälter.

Aufblähbare oder befüllbare Wandbereiche sind optional gleichzeitig Wandbereiche des Schlauchs, wobei erste Wandbereiche jeweils wenigstens drei voneinander getrennte Schichten und zweite Wandbereiche wenigstens zwei voneinander getrennte Schichten jeweils des gummiartigen Stoffes des Wandbereichs des Schlauchs sind. Der Schlauch weist einen ersten Kanal zum Aufblähen oder Befüllen eines ersten Wandbereichs zur Ausbildung eines ersten Ballons in der Harnblase auf. Weiterhin besitzt der Schlauch einen zweiten Kanal zum Befüllen des zweiten Wandbereichs zur Ausbildung einer Wulst in der Harnblase und zum Befüllen eines in Längsrichtung des Schlauchs beabstandet dazu angeordneten ersten Wandbereich zur Ausbildung eines den ersten Ballon umgebenden zweiten Ballons in der Harnblase. Der erste Ballon und der zweite Ballon besitzen jeweils im Querschnitt eine C-Form und das Katheterauge befindet sich im nicht durch die Ballone überdeckten Bereich des Schlauchs. Darüber hinaus weisen die in der Harnröhre angeordnete Wand der Wulst wenigstens einen Durchbruch zur Harnröhre und die in der Harnblase angeordnete Wand des zweiten Ballons wenigstens einen Durchbruch zur Harnblase auf. Der Wandbereich des zweiten Ballons und der Wulst weisen somit jeweils wenigstens einen Durchbruch auf. Diese dienen dem Austritt eines flüssigen Mediums, welches somit in die Harnblase und in die Harnröhre gelangt. Das flüssige Medium kann wenigstens ein Medikament sein oder enthalten. Der zweite Ballon und die Wulst sind so mehrere Reservoirbehälter.

In einer Ausführungsform ist jeweils wenigstens ein entfernbares Fadenstück oder ein entfernbares Drahtstück oder ein entfernbares Garnstück so platziert, dass der Durchbruch ein durch Entfernen des Fadenstücks oder des Drahtstücks oder des Garnstücks ausgebildeter Durchbruch ist.

Die äußere Schicht des Schlauchs ist optional wenigstens bereichsweise eine hydrophobe Schicht und/oder wenigstens bereichsweise eine mit wenigstens einem Stoff imprägnierte Schicht.

In einer Ausführungsform befindet sich unter dem aufgeblähten Wandbereich in Richtung des distalen Endes wenigstens eine Stütze in Röhrchenform. Die Stütze kann aus Draht in Schraubenform oder als Gitter ausgebildet sein.

Die Schichten sind durch Tauchen realisierte Schichten. Zur Erreichung einer gewünschten Dicke und Ausbildung des aufblähbaren Wandbereichs erfolgt ein mehrfaches Tauchen in den flüssigen und sich nach dem mehrfachen Tauchen durch Polymerisation und/oder Vulkanisation ausbildenden gummiartigen Stoff.

Vor Entfernen des Fadens oder des Garns oder des Drahts oder vor Entfernen des Schlauchs vom Stützkörper kann ein entfernbares Trennmittel auf einen Bereich der zweiten Schicht aufgebracht werden. Ein weiterer entfernbarer Faden oder Garn oder Draht wird so platziert, dass dieser am oder auf oder im Trennmittel und außerhalb der zweiten Schicht als distales Ende des Schlauchs endet. Wenigstens eine dritte Schicht eines polymerisierbaren und/oder vulkanisierbaren Stoffes wird auf den Schlauch mit der ersten Schicht und der zweiten Schicht, das entfernbare Trennmittel und den weiteren Faden oder das entfernbare Garn oder den entfernbaren Draht aufgebracht. Die dritte Schicht wird polymerisiert und/oder vulkanisiert. Der Faden oder das Garn oder der Draht und/oder der weitere Faden oder das weitere Garn oder der weitere Draht durch Herausziehen werden oder wird entfernt, so dass Kanäle mit ersten Öffnungen in Richtung der aufblähbaren oder befüllbaren Wandbereiche aus der zweiten und der dritten Schicht über den Trennmitteln und zweiten Öffnungen am distalen Ende des Schlauchs vorhanden sind, so dass die Wandbereiche über die Kanäle aufblähbar oder befüllbar sind. Der Schlauch wird vom Stützkörper entfernt und das Katheterauge wird in den Schlauch eingebracht.

Das Aufbringen der ersten und der zweiten Schicht oder der ersten, der zweiten und der dritten Schicht kann vorteilhafterweise durch Tauchen in den polymerisierbaren und/oder vulkanisierbaren Stoff erfolgen.

Nach Entfernen des wenigstens eines Fadens oder Garns oder Drahts kann das Trennmittel über den Kanal oder die Kanäle entfernt werden.

Ein Ausführungsbeispiel der Erfindung ist in den Zeichnungen jeweils prinzipiell dargestellt und wird im Folgenden näher beschrieben.

Es zeigen
- Fig. 1: ein Blasenkatheter mit einem als Ballon aufgeblähten Wandbereich,
- Fig. 2: ein Blasenkatheter mit einem als Ballon aufgeblähten ersten Wandbereich und einem als Ballon befüllten zweiten Wandbereich,
- Fig. 3: ein Blasenkatheter mit einem als Ballon aufgeblähten Wandbereich und einer befüllten Wulst und
- Fig. 4: ein Blasenkatheter mit als einem Ballon aufgeblähten Wandbereich und als Ballon und Wulst befüllten Wandbereichen.

Ein Blasenkatheter besteht im Wesentlichen aus einem Schlauch 1 mit einer Öffnung 2 am distalen Ende, mindestens einem in der Harnblase 3 zu platzierenden Katheterauge 4 und einem Halter.

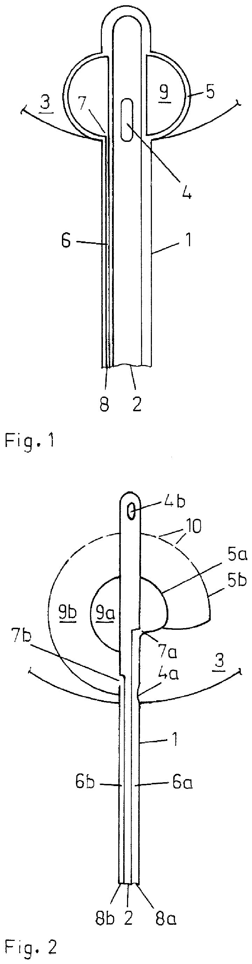

Die Fig. 1 zeigt einen Blasenkatheter mit einem als Ballon 9 aufgeblähten oder befüllten Wandbereich 5 in einer prinzipiellen Darstellung.

Der Endenbereich oder ein Bereich am Ende des Schlauchs 1 besitzt einen aufblähbaren oder befüllbaren Wandbereich 5, so dass im aufgeblähten oder befüllten Zustand ein den Schlauch 1 nicht vollständig umgebender Ballon 9 als Halter des Schlauchs 1 in der Harnblase 3 vorhanden ist. Der Blasenkatheterteil besteht aus mehreren nacheinander aufgebrachten Schichten eines gummiartigen Stoffes. Der aufblähbare oder befüllbare Wandbereich 5 ist wenigstens ein Wandbereich 1 des Schlauchs 1, wobei der Wandbereich 5 wenigstens zwei voneinander getrennte Schichten des gummiartigen Stoffes des Wandbereichs des Schlauchs 1 sind. Ein Bestandteil der Wand des Schlauchs 1 ist weiterhin wenigstens ein Kanal 6 zum Aufblähen und/oder Befüllen und/oder Reinigen des Wandbereichs 5, so dass der Schlauch 1 einstückig ausgebildet ist. Zum Aufblähen oder Befüllen des Wandbereichs 5 weist der Schlauch 1 den Kanal 6 mit einer ersten Öffnung 7 in Richtung aufblähbarem oder befüllbarem Wandbereich 5 und einer zweiten Öffnung 8 am distalen Ende des Schlauchs 1 zum Aufblähen oder Befüllen des Wandbereichs 5 auf. Zur Ausbildung des Kanals 6 ist zwischen Schichten jeweils wenigstens ein entfernbarer Faden oder ein entfernbarer Draht oder ein entfernbares Garn so platziert, dass der Faden oder der Draht oder das Garn in den voneinander getrennten Schichten des Wandbereichs 5 und außerhalb des distalen Endes endet und der Kanal 6 ein durch Entfernen des Fadens oder des Drahts oder des Garns ausgebildeter Kanal 6 ist. Damit ist der Kanal 6 ein Bereich der Wand des Schlauchs 1. Der aufgeblähte oder befüllte Wandbereich 5 ist ein Ballon 9 als Halter mit einem Querschnitt in einer C-Form. Der nicht durch den Ballon 9 überdeckte Bereich des Schlauchs 1 weist das Katheterauge auf. Ein weiteres Katheterauge kann sich über dem Wandbereich 5 in der Harnblase 3 und damit am proximalen Ende des Schlauchs 1 befinden.

Die Fig. 2 zeigt einen Blasenkatheter mit einem als Ballon 9a aufgeblähten ersten Wandbereich 5 und einem als Ballon 9b befüllten zweiten Wandbereich 5 in einer prinzipiellen Darstellung.

In einer Ausführungsform sind aufblähbare oder befüllbare Wandbereiche 5a, 5b gleichzeitig Wandbereiche des Schlauchs 1, wobei die Wandbereiche 5a, 5b jeweils wenigstens drei parallel zueinander angeordneter und voneinander getrennte Schichten des gummiartigen Stoffes des Wandbereichs des Schlauchs 1 sind. Der Schlauch 1 weist wenigstens zwei Kanäle 6a, 6b zum Aufblähen oder Befüllen des jeweiligen Wandbereichs 5a, 5b auf, so dass bei aufgeblähten oder befüllten Wandbereichen 5a, 5b ein erster Ballon 9a in einem zweiten Ballon 9b jeweils im Querschnitt mit einer C-Form vorhanden sind und sich das Katheterauge 4 im nicht durch die Ballone 9a, 9b überdeckten Bereich des Schlauchs 1 befindet. Damit sind im aufgeblähten oder befülltem Zustand den Schlauch 1 nicht vollständig umgebende Ballone 9a, 9b vorhanden. Als Halter des Blasenkatheters dienen dabei beide Ballone 9a, 9b als erster Ballon 9a und zweiter Ballon 9b. Nur bei leerem zweiten Ballon 9b wirkt insbesondere der erste Balbn 9a als Halter des Blasenkatheters. Zum Aufblähen des ersten Ballons insbesondere mit Luft dient der erste Kanal 6a. Mittels des zweiten Kanals 6b wird der zweite Ballon 9b befüllt. Dazu besitzen die Kanäle 6a, 6b als Bestandteile der Wand des Schlauchs 1 erste Öffnungen 7a, 7b zu dem jeweiligen Ballon 9a, 9b und zweite Öffnungen 8a, 8b am distalen Endes des Schlauchs 1 zur Zuführung des jeweiligen Mediums. Der den zweiten Ballon 9b ausbildende zweite Wandbereich 5b besitzt Durchbrüche 10, so dass insbesondere Fluide im zweiten Ballon 9b in die Harnblase gelangen können. Der zweite Ballon 9b stellt somit einen Reservoirbehälter insbesondere für ein Medikament dar.

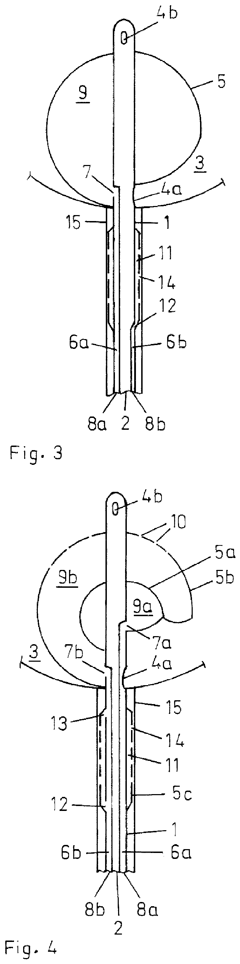

Die Fig. 3 zeigt einen Blasenkatheter mit einem als Ballon 9 aufgeblähten Wandbereich und einer befüllten Wulst 11 in einer prinzipiellen Darstellung.

Ein Blasenkatheter einer weiteren Ausführungsform besitzt einen aufblähbaren und einen befüllbaren Wandbereich 5, die gleichzeitig Wandbereiche des Schlauchs 1 sind. Diese sind jeweils wenigstens zwei voneinander getrennte Schichten des gummiartigen Stoffes des Wandbereichs des Schlauchs 1. Der Schlauch 1 weist zwei Kanäle 6a, 6b zum Aufblähen oder Befüllen eines ersten Wandbereichs 5 und eines in Längsrichtung des Schlauchs 1 beabstandet dazu angeordneten zweiten Wandbereichs auf, so dass bei aufgeblähten oder befüllten ersten Wandbereich 5 ein Ballon 9 im Querschnitt mit einer C-Form in der Harnblase 3 und bei befüllten zweiten Wandbereich eine Wulst 11 in der Harnröhre 15 vorhanden sind. Das Katheterauge 4 befindet sich im nicht durch den Ballon 9 überdeckten Bereich des Schlauchs 1 und ist somit ein Halter des Blasenkatheters. Der Wandbereich der Wulst 11 besitzt Durchbrüche 14 zur Harnröhre 15, so dass darüber Fluid und eventuell wenigstens ein darin enthaltenes Medikament in die Harnröhre 15 gelangen kann.

Die Fig. 4 zeigt einen Blasenkatheter mit als einen Ballon 9a aufgeblähten Wandbereich 5a und als Ballon 9b und Wulst 11 befüllte Wandbereiche 5b, 5c in einer prinzipiellen Darstellung.

Bei einem Blasenkatheter einer weiteren Ausführungsform kann der Schlauch 1 einen dritten aufblähbaren Wandbereich 5c mit einer dritten Öffnung 12 und einer vierten Öffnung 13 zum zweiten Kanal 6b in Verbindung mit dem zweiten Ballon 9b aufweisen, so dass im befüllten Zustand eine den Schlauch 1 wenigstens bereichsweise umgebende Wulst 11 in der Harnröhre 15 angeordnet ist. Der dritte Wandbereich 5c besitzt Durchbrüche 14. Mit Zugabe von Fluid über den zweiten Kanal 6b, gelangt Fluid sowohl in die Wulst 11 als auch in den zweiten Ballon 9b. Über die Durchbrüche 10 des zweiten Wandbereichs 5b des zweiten Ballons 9b kann somit Fluid und damit insbesondere wenigstens ein Medikament in die Harnblase 3 gelangen. Gleichzeitig kann über die Durchbrüche 14 Fluid und damit ebenfalls das Medikament in die Harnröhre 15 gelangen.

In weiteren vorteilhaften Ausgestaltungen der Ausführungsformen kann der Schlauch 1 am proximalen Ende offen sein oder kann in einer weiteren Ausgestaltung bei am proximalen Ende geschlossenem Schlauch 1 ein weiteres Katheterauge als zweites Katheterauge 4b angeordnet sein, wobei das Katheterauge 4 im nicht durch die Ballone 9a, 9b überdeckten Bereich des Schlauchs 1 ein erstes Katheterauge 4a ist.

Der Schlauch 1 des Ausführungsbeipiels, der Ausführungsformen und der Ausgestaltungen besteht dazu aus Schichten die durch Tauchen aufgebracht sind. Zur Erreichung einer gewünschten Dicke und Ausbildung des wenigstens einen aufblähbaren Wandbereichs 5 erfolgt ein mehrfaches Tauchen in den flüssigen und sich nach dem mehrfachen Tauchen durch Polymerisation und/oder Vulkanisation ausbildenden gummiartigen Stoff aus dem dann der Schlauch 1 und damit der jeweilige Blasenkatheter besteht.

### Bezugszeichen

- 1: Schlauch
- 2: Öffnung des Schlauchs
- 3: Harnblase
- 4: Katheterauge
- 5: Wandbereich
- 6: Kanal
- 7: erste Öffnung des ersten und zweiten Kanals
- 8: zweite Öffnung des ersten und zweiten Kanals
- 9: Ballon
- 10: Durchbruch zweiter Wandbereich
- 11: Wulst
- 12: dritte Öffnung des zweiten Kanals
- 13: vierte Öffnung des zweiten Kanals
- 14: Durchbruch dritter Wandbereich
- 15: Harnröhre

## Patentansprüche

1. Blasenkatheter mit einem Schlauch (1) mit einer Öffnung (2) am distalen Ende und mindestens einem in der Harnblase (3) zu platzierenden Katheterauge (4), wobei der Schlauch (1) wenigstens einen gegenüber einem Wandbereich des Schlauchs (1) aufblähbaren oder befüllbaren Wandbereich (5) aufweist, **dadurch gekennzeichnet, dass** der Blasenkatheter aus mehreren nacheinander aufgebrachten Schichten eines gummiartigen Stoffes besteht, dass der Wandbereich (5) wenigstens ein Wandbereich (5) des Schlauchs (1) ist, wobei der Wandbereich (5) wenigstens zwei voneinander getrennte Schichten des gummiartigen Stoffes des Wandbereichs des Schlauchs (1) sind, dass ein Bestandteil der Wand des Schlauchs (1) wenigstens ein Kanal (6) zum Aufblähen und/oder Befüllen und/oder Reinigen des Wandbereichs (5) ist, dass der Schlauch (1) einstückig ausgebildet ist und die miteinander verbundenen Schichten, den wenigstens einen Wandbereich mit den voneinander getrennten Schichten und den Kanal (6) aufweist, dass der aufgeblähte oder befüllte Wandbereich (5) ein den Schlauch (1) wenigstens bereichsweise nicht vollständig umgebender Ballon (9) in der Harnblase (3) ist und dass der nicht durch den Ballon (9) überdeckte Bereich des Schlauchs (1) das Katheterauge (4) aufweist.

2. Blasenkatheter nach Patentanspruch 1, **dadurch gekennzeichnet, dass** der aufgeblähte oder befüllte Wandbereich (5) eine den Schlauch vollständig umgebende Wulst (11) in der Harnröhre (15) ist und dass der Wandbereich (5) wenigstens einen Durchbruch (14) zur Harnröhre (15) aufweist.

3. Blasenkatheter nach Patentanspruch 1 oder 2, **dadurch gekennzeichnet, dass** zwischen Schichten jeweils wenigstens ein entfernbarer Faden oder ein entfernbarer Draht oder ein entfernbares Garn so platziert ist, dass der Faden oder der Draht oder das Garn in den voneinander getrennten Schichten des Wandbereichs (5) und außerhalb des distalen Endes endet und der Kanal (6) ein durch Entfernen des Fadens oder des Drahts oder des Garns ausgebildeter Kanal (6) ist.

4. Blasenkatheter nach einem der Patentansprüche 1 bis 3, **dadurch gekennzeichnet, dass** aufblähbare oder befüllbare Wandbereiche (5) gleichzeitig Wandbereiche des Schlauchs (1) sind, wobei die Wandbereiche (5) jeweils wenigstens drei voneinander getrennte Schichten des gummiartigen Stoffes des Wandbereichs des Schlauchs (1) sind, dass der Schlauch (1) wenigstens zwei Kanäle (6a, 6b) zum Aufblähen oder Befüllen des jeweiligen Wandbereichs (5a, 5b) aufweist, so dass bei aufgeblähten oder befüllten Wandbereichen (5a, 5b) ein erster Ballon (9a) in einem zweiten Ballon (9b) jeweils im Querschnitt mit einer C-Form vorhanden sind und sich das Katheterauge (4) im nicht durch die Ballone (9a, 9b) überdeckten Bereich des Schlauchs (1) befindet, und dass die in der Harnblase (3) angeordnete Wanddes zweiten Ballons (9b) wenigstens einen Durchbruch (10) zur Harnblase (3) aufweist.

5. Blasenkatheter nach einem der Patentansprüche 1 bis 4, **dadurch gekennzeichnet, dass** aufblähbare oder befüllbare Wandbereiche (5) gleichzeitig Wandbereiche des Schlauchs (1) sind, wobei die Wandbereiche (5) wenigstens zwei voneinander getrennte Schichten des gummiartigen Stoffes des Wandbereichs des Schlauchs (1) sind, dass der Schlauch (1) zwei Kanäle (6a, 6b) zum Aufblähen oder Befüllen eines ersten Wandbereichs (5) und eines in Längsrichtung des Schlauchs (1) beabstandet dazu angeordneten zweiten Wandbereichs aufweist, so dass bei aufgeblähten oder befüllten ersten Wandbereich (5) ein Ballon (9) im Querschnitt mit einer C-Form in der Harnblase (3) und bei befüllten zweiten Wandbereich eine Wulst (11) in der Harnröhre (15) vorhanden sind, wobei sich das Katheterauge (4) im nicht durch den Ballon (9) überdeckten Bereich des Schlauchs (1) befindet, und dass der Wandbereich der Wulst (11) wenigstens einen Durchbruch (14) zur Harnröhre (15) aufweist.

6. Blasenkatheter nach einem der Patentansprüche 1 bis 5, **dadurch gekennzeichnet, dass** aufblähbare oder befüllbare Wandbereiche (5) gleichzeitig Wandbereiche des Schlauchs (1) sind, wobei erste Wandbereiche (5) jeweils wenigstens drei voneinander getrennte Schichten und zweite Wandbereiche wenigstens zwei voneinander getrennte Schichten jeweils des gummiartigen Stoffes des Wandbereichs des Schlauchs (1) sind, dass der Schlauch (1) einen ersten Kanal (6a) zum Aufblähen oder Befüllen eines ersten Wandbereichs (5) zur Ausbildung eines ersten Ballons (9a) in der Harnblase (3) aufweist, dass der Schlauch (1) einen zweiten Kanal (6b) zum Befüllen des zweiten Wandbereichs zur Ausbildung einer Wulst (11) in der Harnblase (15) und zum Befüllen eines in Längsrichtung des Schlauchs (1) beabstandet dazu angeordneten ersten Wandbereich (5) zur Ausbildung eines den ersten Ballon (9a) umgebenden zweiten Ballons (9b) in der Harnblase (3) aufweist, dass der erste Ballon (9a) und der zweite Ballon (9b) jeweils im Querschnitt eine C-Form besitzen und sich das Katheterauge (4) im nicht durch die Ballone (9a, 9b) überdeckten Bereich des Schlauchs (1) befindet und dass die in der Harnröhre (15) angeordnete Wand der Wulst (11) wenigstens einen Durchbruch (14) zur Harnröhre (15) und die in der Harnblase 3 angeordnete Wand des zweiten Ballons (9b) wenigstens einen Durchbruch (10) zur Harnblase (3) aufweisen.

7. Blasenkatheter nach Patentanspruch 2, 4, 5 oder 6, **dadurch gekennzeichnet, dass** jeweils wenigstens ein entfernbares Fadenstück oder ein entfernbares Drahtstück oder ein entfernbares Garnstück so platziert ist, dass der Durchbruch (10, 14) ein durch Entfernen des Fadenstücks oder des Drahtstücks oder des Garnstücks ausgebildeter Durchbruch (10, 14) ist.

8. Blasenkatheter nach einem der Patentansprüche 1 bis 7, **dadurch gekennzeichnet, dass** die äußere Schicht des Schlauchs (1) wenigstens bereichsweise eine hydrophobe Schicht ist und/oder dass die äußere Schicht des Schlauchs (1) wenigstens bereichsweise eine mit wenigstens einem Stoff imprägnierte Schicht ist.

9. Blasenkatheter nach einem der Patentansprüche 1 bis 8, **dadurch gekennzeichnet, dass** unter dem aufgeblähten Wandbereich (5) in Richtung des distalen Endes wenigstens eine Stütze in Röhrchenform angeordnet ist.

10. Blasenkatheter nach einem der Patentansprüche 1 bis 9, **dadurch gekennzeichnet, dass** die Schichten durch mehrmaliges Tauchen in ein Reservoir des sich ausbildenden gummiartigen Stoffes realisierte Schichten sind.

11. Verfahren zur Herstellung eines Blasenkatheters mit einem Schlauch (1) mit einer Öffnung (2) am distalen Ende und mindestens einem in der Harnblase (3) zu platzierenden Katheterauge (4) mit den folgenden Schritten:
- Aufbringen wenigstens einer ersten Schicht eines polymerisierbaren und/oder vulkanisierbaren Stoffes auf einen Stützkörper,
- Polymerisieren und/oder Vulkanisieren der Schicht zu einem einseitig verschlossenen Schlauch (1) eines gummiartigen Stoffs,
- Aufbringen eines entfernbaren Trennmittels auf einen Bereich des Schlauchs (1),
- Platzieren eines entfernbaren Fadens oder Garns oder Drahts, so dass dieser am oder auf oder im Trennmittel und außerhalb des Schlauchs (1) als distales Ende endet,
- Aufbringen wenigstens einer zweiten Schicht eines polymerisierbaren und/oder vulkanisierbaren Stoffes auf den Schlauch (1), das entfernbare Trennmittel und den Faden oder das Garn oder den Draht,
- Polymerisieren und/oder Vulkanisieren der zweiten Schicht,
- Entfernen des Fadens oder Garns oder Drahts durch Herausziehen, so dass ein Kanal (6) mit einer ersten Öffnung (7) in Richtung aufblähbaren oder befüllbaren Wandbereich (5) aus der zweiten Schicht über dem Trennmittel und einer zweiten Öffnung (8) am distalen Ende des Schlauchs (1) vorhanden ist, so dass der Wandbereich (5) über den Kanal (6) aufblähbar oder befüllbar ist,
- Entfernen des Schlauchs (1) vom Stützkörper und
- Einbringen des Katheterauges (4) in den Schlauch (1).

12. Verfahren nach Patentanspruch 11, **dadurch gekennzeichnet,**
- **dass** vor Entfernen des Fadens oder des Garns oder des Drahts oder vor Entfernen des Schlauchs (1) vom Stützkörper ein entfernbares Trennmittel auf einen Bereich der zweiten Schicht aufgebracht wird,
- **dass** ein weiterer entfernbarer Faden oder Garn oder Draht so platziert wird, dass dieser am oder auf oder im Trennmittel und außerhalb der zweiten Schicht als distales Ende des Schlauchs (1) endet,
- **dass** wenigstens eine dritte Schicht eines polymerisierbaren und/oder vulkanisierbaren Stoffes auf den Schlauch (1) mit der ersten Schicht und der zweiten Schicht, das entfernbare Trennmittel und den weiteren Faden oder das entfernbare Garn oder den entfernbaren Draht aufgebracht wird,
- **dass** die dritte Schicht polymerisiert und/oder vulkanisiert wird,
- **dass** der Faden oder das Garn oder der Draht und/oder der weitere Faden oder das weitere Garn oder der weitere Draht durch Herausziehen entfernt werden oder entfernt wird, so dass Kanäle (6a, 6b) mit ersten Öffnungen (7a, 7b) in Richtung der aufblähbaren oder befüllbaren Wandbereiche (5a, 5b) aus der zweiten und der dritten Schicht über den Trennmitteln und zweiten Öffnungen (8a, 8b) am distalen Ende des Schlauchs (1) vorhanden sind, so dass die Wandbereiche (5a, 5b) über die Kanäle (6a, 6b) aufblähbar oder befüllbar sind,
- **dass** der Schlauchs (1) vom Stützkörper entfernt wird und
- **dass** das Katheterauge (4) in den Schlauch (1) eingebracht wird.

13. Verfahren nach Patentanspruch 11 oder 12, **dadurch gekennzeichnet, dass** das Aufbringen der ersten und der zweiten Schicht oder der ersten, der zweiten und der dritten Schicht durch Tauchen in den polymerisierbaren und/oder vulkanisierbaren Stoff erfolgt.

14. Verfahren nach einem der Patentansprüche 11 bis 13, **dadurch gekennzeichnet, dass** nach Entfernen des wenigstens eines Fadens oder Garns oder Drahts das Trennmittel über den Kanal (6) oder die Kanäle entfernt wird.
